# EUROPEAN PATENT SPECIFICATION

(11) **EP 1 561 824 B1**
(45) Date of publication and mention of the grant of the patent: **22.04.2009**
(21) Application number: 05002102.1
(22) Date of filing: 02.02.2005
(51) Int. Cl.: C12Q 1/68

(54) **Norovirus detection reagent**
Nachweisreagenz für Noroviridae
Agent pour la détection des Noroviridae

(30) Priority: 04.02.2004 JP 2004027802
(43) Date of publication of application: 10.08.2005
(60) Divisional application: 07017946.0
(73) Proprietor: TOSOH CORPORATION, Shunan-shi Yamaguchi-ken 746-8501 (JP)
(72) Inventor: Masuda, Noriyoshi, Tokyo (JP); Yasukawa, Kiyoshi, Kawasaki-shi, Kanagawa (JP); Horie, Ryuichi, Zama-shi, Kanagawa (JP)
(74) Representative: Pautex Schneider, Nicole Véronique

(56) References cited:
- EP-A- 1 329 523
- YAN HAINIAN ET AL: "Detection of norovirus (GI, GII), Sapovirus and astrovirus in fecal samples using reverse transcription single-round multiplex PCR." JOURNAL OF VIROLOGICAL METHODS, vol. 114, no. 1, December 2003 (2003-12), pages 37-44, XP008046217 ISSN: 0166-0934
- KAGEYAMA TSUTOMU ET AL: "Broadly reactive and highly sensitive assay for Norwalk-like viruses based on real-time quantitative reverse transcription-PCR." JOURNAL OF CLINICAL MICROBIOLOGY. APR 2003, vol. 41, no. 4, April 2003 (2003-04), pages 1548-1557, XP002326299 ISSN: 0095-1137
- HOEHNE M ET AL: "Detection and characterization of norovirus outbreaks in Germany: Application of a one-tube RT-PCR using a fluorogenic real-time detection system." JOURNAL OF MEDICAL VIROLOGY, vol. 72, no. 2, December 2003 (2003-12), pages 312-319, XP002326300 ISSN: 0146-6615
- DATABASE EMBL [Online] 13 October 2000 (2000-10-13), "Norwalk-like virus strain Rob546-02/99-QC RNA polymerase gene, partial cds." XP002326301 retrieved from EBI accession no. EM_VI:AF305593 Database accession no. AF305593
- DATABASE EMBL [Online] 26 September 2001 (2001-09-26), "Norwalk virus gene for capsid protein, partial cds, strain:NV/Saitama KU115cGI/99/JP." XP002326302 retrieved from EBI accession no. EM_VI:AB058524 Database accession no. AB058524 & KAGEYAMA TSUTOMU ET AL: "Broadly reactive and highly sensitive assay for Norwalk-like viruses based on real-time quantitative reverse transcription-PCR." JOURNAL OF CLINICAL MICROBIOLOGY. APR 2003, vol. 41, no. 4, April 2003 (2003-04), pages 1548-1557, ISSN: 0095-1137
- DATABASE EMBL [Online] 26 September 2001 (2001-09-26), "Norwalk virus gene for capsid protein, partial cds, strain:NV/Saitama KU83bGI/99/JP." XP002326303 retrieved from EBI accession no. EM_VI:AB058546 Database accession no. AB058546
- DATABASE EMBL [Online] 10 October 2001 (2001-10-10), "Norwalk-like virus genogroup 2 isolate 86/2001 capsid protein gene, partial cds." XP002326304 retrieved from EBI accession no. EM_VI:AF408853 Database accession no. AF408853
- KARIM MOHAMMAD R ET AL: "Detection of noroviruses in water: Summary of an international workshop." JOURNAL OF INFECTIOUS DISEASES, vol. 189, no. 1, 1 January 2004 (2004-01-01), pages 21-28, XP008046225 ISSN: 0022-1899
- UYTTENDAELE M. ET AL: 'Development of NASBA, a nucleic acid amplification system, for identification of Listeria monocytogenes and comparison to ELISA and a modified FDA method' INT. JOURNAL OF FOOD MICROBIOLOGY vol. 27, no. 1, 1995, pages 77 - 89
- LOEFFLER J. ET AL: 'Nucleic Acid Sequence-Based Amplification of Aspergillus RNA in Blood Samples' J. CLINICAL MICROBIOLOGY vol. 39, no. 4, 2001, pages 1626 - 1629
- JEAN J. ET AL: 'Detection of Hepatits A Virus by the Nucliec Acid Sequence-Based Amplification Technique and Comparison with Reverse Transcriptase-PCR' APPL. ENVIRON. MICROBIOLOGY vol. 67, no. 12, 2001, pages 5593 - 5600
- JEAN J. ET AL: 'Multiplex Nucleic Acid Sequence Based Amplification for Simultaneous Detection of Several Enteric Viruses in Model Ready-To-Eat Foods' APPL. ENVIRON. MICROBIOLOGY vol. 70, no. 11, November 2004, pages 6603 - 6610
- MOORE C. ET AL: 'Evaluation of a broadly reactive nucleic acid sequence based amplification assay for the detection of noroviruses in faecal materkal' J. CLINIC. VIROLOGY vol. 29, no. 4, April 2004, pages 290 - 296
- RUTJES S.A. ET AL: 'Real-Time Detection of Noroviruses in Surface Water by Use of a Broadly Reactive Nucleic Acid Sequence-Based Amplification Assay' APPL. ENVIRONM. MICROBIOLOGY vol. 72, no. 8, August 2006, pages 5349 - 5358

## Description

### Field of the Invention

Norovirus is known to be a virus that typically causes viral food poisoning. The present invention relates to a detection method for detecting norovirus in clinical examinations, public health examinations, food evaluations and food poisoning examinations.

### Prior Art

Norovirus is a member of the human calicivirus family, and has a genome consisting of a single-strand RNA of about 7000 bases. Norovirus is also referred to as Small Round Structured Virus (SRSV).

Roughly 20% of the cases of food poisoning reported in Japan are estimated to be caused by viruses. Norovirus is detected in about 80% of these cases of viral food poisoning. The main infection source is food, and raw oysters are frequently the problem. In addition, norovirus has also been detected in (sporadic) acute gastroenteritis among infants, and the possibility of person-to-person propagation has been suggested. Since testing for norovirus is therefore an important issue in terms of public health and food quality control, there is a need for the development of a highly-sensitive and rapid testing method capable of detecting all or most of the subtypes, using a gene amplification process. In contrast, detection of norovirus is currently based on observation by electron microscopy. Although this method makes it possible to detect all subtypes, because detection requires an amount of virus of 10⁶ cells/mL or more and the sensitivity of the test is low, specimens are limited to patient stool samples.

### Disclosure of the Invention

Norovirus is broadly divided into two types consisting of genogroup I (GI) and genogroup II (GII) according to its genotype. Moreover, GI is classified, on the basis of the genotype, into a plurality of subtypes typically represented by Chiba, Desert Shield, Norwalk and Southampton, while GII is classified, on the basis of the genotype, into a plurality of subtypes typically represented by Camberwell, Hawaii, Mexico and Snow Mountain. The base sequence homology among subtypes belonging to GI and among subtypes belonging to GII is about 70%. In addition, the homology between base sequences of GI and GII is about 40-50%.

In order to improve the detectability of a norovirus detection reagent used in a gene amplification process, regarding the regions to be used for primer bindings, there must be at least two regions in a length of at least 20 bases, each region having a base sequence which is common among all subtypes. However, having researched the homology of the following six sequences indicated as norovirus sequences in GenBank (Chiba (No. AB042808), Norwalk (No. NC_001959), Southampton (No. L07418), Camberwell (No. AF145896), Hawaii (No. U07611) and HuCV (No. AY032605)), there are no region of 20 or more bases in which the base sequence is the same among all subtypes.

Under these circumstances, the inventors of the present invention had previously provided norovirus detection methods (Japanese Unexamined Patent Publication No. 2002-51778, Japanese Unexamined Patent Publication No. 2002-153289, Japanese Unexamined Patent Publication No. 2002-218999). However, as the primer base sequences are designed on the basis of the base sequence of each subtype, it is not possible to detect other subtypes. In addition, a norovirus detection reagent using PCR disclosed in Japanese Unexamined Patent Publication No. 2000-300297 can only be used to detect the Norwalk and Snow Mountain subtypes.

The publication of H. Yan et al., in Journal of Virological Methods 114 (2003) 37-44, discloses a method for the detection of norovirus (GI, GII) in fecal samples using reverse transcription single-rounds multiplex PCR.

The publication of T. Kageyama et al., in Journal of Clinical Microbiology 41 (2003) 1548-1557, discloses a method for the detection of Norwalk-like viruses using real-time quantitative Reverse Transcription-PCR (RT-PCR).

EP-A-1 329 523 discloses a method for the detection of Norwalk-like virus (GII) using RT-PCR.

The publication of M. Höhne et al., in Journal of Medical Virology 72 (2004) 312-319 discloses a method for the detection of norovirus using one-tube RT-PCR.

In order to solve this problem, the inventors of the present invention developed a norovirus detection method that detects all subtypes of GI by analyzing the base sequences of the norovirus registered in GenBank as well as the norovirus base sequences determined on their own, and further determining the primer binding regions from these sequences.

The invention of the present application provides a detection method for detecting norovirus of the genogroup I (GI), using an RNA amplification process comprising the steps of:
producing a DNA with an RNA-dependent DNA polymerase using a specific sequence of norovirus genome RNA as a template, as well as a first primer having a sequence homologous to said specific sequence, and a second primer having a sequence complementary to said specific sequence, wherein either the first primer or second primer has a sequence in which a promoter sequence of an RNA polymerase has been added to its 5' end, thereby forming a double-strand RNA-DNA;
degrading the RNA portion of said double-strand RNA-DNA by ribonuclease H, thereby producing a single-strand DNA; and
producing a double-strand DNA having said promoter sequence capable of transcribing the RNA composed of the specific sequence of the RNA or the sequence complementary to said specific sequence of the RNA with a DNA-dependent DNA polymerase using said single-strand DNA as a template;
wherein, the double-strand DNA produces an RNA transcription product in the presence of the RNA polymerase, and said RNA transcription product serves as a template for the subsequent cDNA synthesis with the RNA-dependent DNA polymerase;
**characterized in that**
the first primer is an oligonucleotide consisting of at least the sequence shown in SEQ. ID No. 1 ; and
the second primer is an oligonucleotide consisting of at least the sequence shown in SEQ. ID No. 4 or 20.

Preferably, the aforementioned RNA amplification process is carried out in the presence of a cleaving oligonucleotide that cleaves the aforementioned target RNA at the 5' end of the aforementioned specific sequence and has a sequence complementary to the region adjacent to and overlapping with the 5' end of said specific sequence.

Preferably, the aforementioned first primer is an oligonucleotide consisting of the sequence shown in SEQ. ID No. 1.

Moreover, the aforementioned second primer is preferably an oligonucleotide consisting of the sequence shown in SEQ. ID No. 4 or 20.

In another preferable embodiment, the aforementioned RNA amplification process is carried out in the presence of an oligonucleotide labeled with an intercalator fluorescent pigment, and the detection of norovirus is carried out by measuring the fluorescent intensity of the reaction solution. Here, the sequence of said oligonucleotide is complementary to at least a portion of the sequence of the aforementioned RNA transcription product and, in the situation where complementary binding of said oligonucleotide to said RNA transcription product occurs, the fluorescent properties of the reaction solution change in comparison with the situation where no complex is formed.

Preferably, the aforementioned oligonucleotide labeled with an intercalator fluorescent pigment consists of at least the sequence shown in SEQ. ID No. 5. The following provides a detailed explanation of the present invention.

### Best Mode for Carrying Out the Invention

In the present invention, an oligonucleotide consisting of the sequences shown in SEQ. ID Nos. 1, 4 and 20 are used as a primer. Here, SEQ. ID Nos. 4 and 20 are regions that are close to each other. Thus, in the case of carrying out PCR, for example, an example of combinations includes a combination of SEQ. ID No. 1 and SEQ. ID No. 4 or 20. This applies similarly to other DNA amplification methods other than PCR.

The present invention includes, for example, the NASBA method, 3SR method or TRC method (see, e.g., Japanese Unexamined Patent Publication No. 2000-014400), which amplifies an RNA sequence of norovirus by the concerted action of reverse transcriptase and RNA polymerase (by reacting them under condition by which the reverse transcriptase and RNA polymerase act in concert). Here, although there are no particular limitations on temperature, it is preferably 35 to 50°C.

In the aforementioned embodiment of the invention of the present application, it is necessary for a target RNA to be cleaved at the 5' end of a specific sequence. A preferable method for cleaving the target RNA in this manner preferably consists of cleaving the target RNA with ribonuclease H or the like by adding an oligonucleotide having a sequence complementary to the region adjacent to and overlapping with the 5' end of the specific sequence (cleaving oligonucleotide). In said cleaving oligonucleotide, the 3' end hydroxyl group is preferably chemically modified, for example, aminated, in order to suppress an elongation reaction from the 3' end.

Although the amplification product obtained in the aforementioned nucleic acid amplification method can be detected with a known nucleic acid detection method, in a preferable embodiment, the aforementioned nucleic acid amplification is preferably carried out in the presence of an oligonucleotide labeled with an intercalator fluorescent pigment followed by measurement of the change in the fluorescent properties of the reaction solution. In said oligonucleotide, as the intercalator fluorescent pigment is bound to the phosphorous atom in the oligonucleotide by means of a linker, the intercalator portion that forms a double strand with the target nucleic acid (complementary nucleic acid) intercalates to the double strand portion resulting in a change in fluorescent properties, thereby resulting in the characteristic of not requiring separation and analysis (Ishiguro, T. et al. (1996) Nucleic Acid Res. 24 (24) 4992-4997).

The sequence bound by said oligonucleotide may be any sequence that is amplified in the genome RNA of norovirus, and although there are no particular limitations thereon, a sequence consisting of at least the sequence shown in SEQ. ID No. 5 is preferable. In addition, the hydroxyl group at the 3' end of said oligonucleotide is preferably chemically modified (such as by an addition of glycolic acid) to suppress the elongation reaction which may occur by using said oligonucleotide as a primer.

As a result, norovirus RNA can be amplified and detected in a single tube, at a constant temperature and in a single step, rapidly and with high sensitivity, thereby facilitating application to automation.

The detection method of the invention of the present application is useful for detecting all GI subtypes of norovirus both rapidly and with high sensitivity.

Although the following provides a more detailed explanation of the invention of the present application through examples, the present invention is not limited by these examples.

### Example 1 (Forming part of the invention only when GI is concerned)

In order to show that combination (a) shown in Table 1 is capable of detecting all subtypes of GI and that combinations (b) and (c) not forming part of the present invention are capable of detecting all subtypes of GII, DNA (hereafter, referred to as "artificial standard DNA)" and RNA (hereafter, referred to as "artificial standard RNA") respectively corresponding to four subtypes of GI and four subtypes of GII were prepared using the methods shown in (1) to (8) below. The artificial standard RNA was measured using the methods shown in (9) to (12).
(1) Artificial standard DNA was designed so as to contain an oligonucleotide binding region used in combinations (a) through (c) based on the base sequence of the Chiba subtype of norovirus (GenBank No. AB042808), and then artificial standard RNA consisting of 90 bases (NV1ART-C, SEQ. ID No. 6) was prepared by in vitro transcription. This standard RNA was used as the sample. After quantifying it by UV absorption at 260 nm, the RNA sample was diluted to 10⁶ copies/5 µL with an RNA diluent (10 mM Tris-HCl (pH 8.0), 1 mM EDTA, 5 mM DTT, 0.25 U/µL RNase inhibitor (Takara Bio)).
(2) Artificial standard DNA was designed so as to contain an oligonucleotide binding region used in combinations (a) through (c) based on the base sequence of the Desert Shield subtype of norovirus (GenBank No. U04469), and then artificial standard RNA consisting of 90 bases (NV1ART-D, SEQ. ID No. 7) was prepared by in vitro transcription. This standard RNA was used as the sample. After quantifying it by UV absorption at 260 nm, the RNA sample was diluted to 10⁶ copies/5 µL with an RNA diluent (10 mM Tris-HCl (pH 8.0), 1 mM EDTA, 5 mM DTT, 0.25 U/µL RNase inhibitor (Takara Bio)).
(3) Artificial standard DNA was designed so as to contain an oligonucleotide binding region used in combinations (a) through (c) based on the base sequence of the Norwalk subtype of norovirus (GenBank No. NC 001959), and then artificial standard RNA consisting of 90 bases (NV1ART-N, SEQ. ID No. 8) was prepared by in vitro transcription. This standard RNA was used as the sample. After quantifying it by UV absorption at 260 nm, the RNA sample was diluted to 10⁶ copies/5 µL with an RNA diluent (10 mM Tris-HCl (pH 8.0), 1 mM EDTA, 5 mM DTT, 0.25 U/µL RNase inhibitor (Takara Bio)).
(4) Artificial standard DNA was designed so as to contain an oligonucleotide binding region used in combinations (a) through (c) based on the base sequence of the Southampton subtype of norovirus (GenBank No. L07418), and then artificial standard RNA consisting of 90 bases (NV1ART-S, SEQ. ID No. 9) was prepared by in vitro transcription. This standard RNA was used as the sample. After quantifying it by UV absorption at 260 nm, the RNA sample was diluted to 10⁶ copies/5 µL with an RNA diluent (10 mM Tris-HCl (pH 8.0), 1 mM EDTA, 5 mM DTT, 0.25 U/µL RNase inhibitor (Takara Bio)).
(5) Artificial standard DNA was designed so as to contain an oligonucleotide binding region used in combinations (a) through (c) based on the base sequence of the Camberwell subtype of norovirus (GenBank No. AF145896), and then artificial standard RNA consisting of 90 bases (NV2ART-C, SEQ. ID No. 10) was prepared by in vitro transcription. This standard RNA was used as the sample. After quantifying it by UV absorption at 260 nm, the RNA sample was diluted to 10⁶ copies/5 µL with an RNA diluent (10 mM Tris-HCl (pH 8.0), 1 mM EDTA, 5 mM DTT, 0.25 U/µL RNase inhibitor (Takara Bio)).
(6) Artificial standard DNA was designed so as to contain an oligonucleotide binding region used in combinations (a) through (c) based on the base sequence of the Hawaii subtype of norovirus (GenBank No. U07611), and then artificial standard RNA consisting of 90 bases (NV2ART-H, SEQ. ID No. 11) was prepared by in vitro transcription. This standard RNA was used as the sample. After quantifying it by UV absorption at 260 nm, the RNA sample was diluted to 10⁶ copies/5 µL with an RNA diluent (10 mM Tris-HCl (pH 8.0), 1 mM EDTA, 5 mM DTT, 0.25 U/µL RNase inhibitor (Takara Bio)).
(7) Artificial standard DNA was designed so as to contain an oligonucleotide binding region used in combinations (a) through (c) based on the base sequence of the Mexico subtype of norovirus (GenBank No. U22498), and then artificial standard RNA consisting of 90 bases (NV2ART-M, SEQ. ID No. 12) was prepared by in vitro transcription. This standard RNA was used as the sample. After quantifying it by UV absorption at 260 nm, the RNA sample was diluted to 10⁶ copies/5 µL with an RNA diluent (10 mM Tris-HCl (pH 8.0), 1 mM EDTA, 5 mM DTT, 0.25 U/µL RNase inhibitor (Takara Bio)).
(8) Artificial standard DNA was designed so as to contain an oligonucleotide binding region used in combinations (a) through (c) based on the base sequence of the Snow Mountain subtype of norovirus, and then artificial standard RNA consisting of 90 bases (NV2ART-S, SEQ. ID No. 13) was prepared by in vitro transcription. This standard RNA was used as the sample. After quantifying it by UV absorption at 260 nm, the RNA sample was diluted to 10⁶ copies/5 µL with an RNA diluent (10 mM Tris-HCl (pH 8.0), 1 mM EDTA, 5 mM DTT, 0.25 U/µL RNase inhibitor (Takara Bio)).
(9) 20 µL of a reaction solution having the composition indicated below were dispensed into 0.5 mL PCR tubes (Individual Dome Cap PCR Tubes, SSI) followed by the addition of 5 µL of the RNA samples prepared in (1) through (8) thereto. Furthermore, solutions were prepared so that the combinations of the first primer, the second primer and the cleaving oligonucleotide were combined as shown in Table 1.

### Composition of Reaction Solution (concentrations are shown as the concentration in the final reaction solution volume of 30 µL)

60 mM Tris-HCl buffer (pH 8.6)
17 mM magnesium chloride
120 mM potassium chloride
6 U RNase inhibitor
1 mM DTT
0.25 mM each of dATP, dCTP, dGTP and dTTP
3.6 mM ITP
3.0 mM each of ATP, CTP, GTP and UTP
0.16 µM cleaving oligonucleotide
1.0 µM first primer
1.0 µM second primer
25 nM oligonucleotide labeled with intercalator pigment (YO-NV-S-G, SEQ ID. No. 5; labeled with the intercalator fluorescent pigment between the 12th "C" and 13th "A" from the 5' end, and the hydroxyl group on its 3' end being modified with a glycol group.)
13% DMSO

### Distilled water for adjusting volume

(10) After incubating the aforementioned reaction solution at 43°C for 2 minutes, 5 µL of an enzyme solution having the composition indicated below and pre-heated for 2 minutes at 43°C were added.

### Composition of Enzyme Solution (values shown indicate the values for a final reaction solution volume of 30 µL)

2.0% sorbitol
3.6 µg bovine serum albumin
142 U T7 RNA polymerase (Invitrogen)
6.4 U AMV reverse transcriptase (LifeScience)

### Distilled water for adjusting volume

(11) Subsequently, the reaction solution in each of the PCR tubes was measured, over time, at an excitation wavelength of 470 nm and fluorescent wavelength of 520 nm, while being incubated at 43°C, using a fluorescent spectrophotometer equipped with a temperature control function and capable of directly measuring the PCR tubes.
(12) The results for the rise time (the time required for the ratio in the fluorescence increase to reach 1.2 times the sum of the negative control sample's average value plus 3 standard deviations) of the norovirus artificial standard RNA when using each oligonucleotide combination are shown in Table 2. Four standard RNA samples of GI were shown to be detected within 30 minutes with combination (a), while four standard RNA samples of GII were shown to be detected within 30 minutes with combinations (b) and (c).

**Table 1**

| Combination | 1st oligo. | 1st oligo. | 3rd oligo. |
|---|---|---|---|
| (a) | NV1-3SM-9 | NV1-3FM-9 | NV2-7RM |
| (b) | NV2-3SM | NV2-3FM | NV2-7RM |
| (c) | NV2-3S20+3 | NV2-3F20+3 | NV2-7RM |

Table 1 shows the combinations of the first primer, the second primer and the cleaving oligonucleotide used in the experimental system. The hydroxyl group on the 3' end of the cleaving oligonucleotide base sequence was aminated in the combinations of oligonucleotides for detecting norovirus.

The region from the 1st "A" to the 22nd "A" from the 5' end of the base sequence of the first primer is a T7 promoter region, and the subsequent region from the 23rd "G" to the 28th "A" is an enhancer sequence. Furthermore, combination (a) is designed using mainly GI norovirus as the target, while combinations (b) and (c) not forming part of the present invention are designed using mainly GII norovirus as the target.

Cleaving oligonucleotides:
NV1-3SM-9 (SEQ. ID No. 14)
NV2-3SM (SEQ. ID No. 15)
NV2-3S20+3 (SEQ. ID No. 16)

First primer:
NV1-3FM-9 (SEQ. ID No. 17)
NV2-3FM (SEQ. ID No. 18)
NV2-3F20+3 (SEQ. ID No. 19)

Second primer:
NV2-7RM (SEQ. ID No. 4)

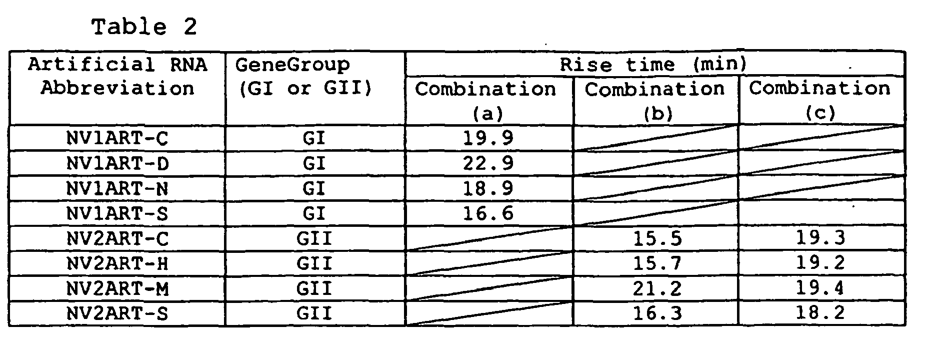

Table 2 shows the results of measuring norovirus artificial standard RNA (four samples each for GI and GII) using the combinations of oligonucleotides shown in Table 1. Among the combinations of oligonucleotides shown in Table 1, combination (a) detected the four standard RNA samples of GI within 30 minutes, while combinations (b) and (c) not forming part of the present invention detected the four standard RNA samples of GII within 30 minutes.

### Example 2

Artificial standard RNA of four GI subtypes were measured according to the methods shown in (1) through (8) below in order to show that one of the combinations of oligonucleotides of the invention of the present application is capable of detecting all subtypes of GI.
(1) The Chiba subtype of artificial standard RNA (NV1ART-C, SEQ. ID No. 6) was used as the sample in the same manner as Example 1. After quantifying it by UV absorption at 260 nm, the RNA was diluted to 10⁶ copies/5 µL with an RNA diluent (10 mM Tris-HCl (pH 8.0), 1 mM EDTA, 5 mM DTT, 0.25 U/µL RNase inhibitor (Takara Bio)).
(2) The Desert Shield subtype of artificial standard RNA (NV1ART-D, SEQ. ID No. 7) was used as the sample in the same manner as Example 1. After quantifying it by UV absorption at 260 nm, the RNA was diluted to 10⁶ copies/5 µL with an RNA diluent (10 mM Tris-HCl (pH 8.0), 1 mM EDTA, 5 mM DTT, 0.25 U/µL RNase inhibitor (Takara Bio)).
(3) The Norwalk subtype of artificial standard RNA (NV1ART-N, SEQ. ID No. 8) was used as the sample in the same manner as Example 1. After quantifying it by UV absorption at 260 nm, the RNA was diluted to 10⁶ copies/5 µL with an RNA diluent (10 mM Tris-HCl (pH 8.0), 1 mM EDTA, 5 mM DTT, 0.25 U/µL RNase inhibitor (Takara Bio)).
(4) The Southampton subtype of artificial standard RNA (NV1ART-S, SEQ. ID No. 9) was used as the sample in the same manner as Example 1. After quantifying it by UV absorption at 260 nm, the RNA was diluted to 10⁶ copies/5 µL with an RNA diluent (10 mM Tris-HCl (pH 8.0), 1 mM EDTA, 5 mM DTT, 0.25 U/µL RNase inhibitor (Takara Bio)).
(5) 20 µL of a reaction solution having the composition indicated below were dispensed into 0.5 mL PCR tubes (Individual Dome Cap PCR Tubes, SSI) followed by the addition of 5 µL of the RNA samples prepared in (1) through (4) thereto.

### Composition of Reaction Solution (concentrations are shown as the concentration in the final reaction solution volume of 30 µL)

60 mM Tris-HCl buffer (pH 8.6)
17.2 mM magnesium chloride
120 mM potassium chloride
6 U RNase inhibitor
1 mM DTT
0.25 mM each of dATP, dCTP, dGTP and dTTP
3.6 mM ITP
3.0 mM each of ATP, CTP, GTP and UTP
0.16 µM cleaving oligonucleotide (NV1-3SM-9, SEQ. ID No. 14, hydroxyl group on 3' end being aminated)
1.0 µM first primer (NV1-3FM-9, SEQ. ID No. 17)
1.0 µM second primer (NV1-7RM18-1, SEQ. ID No. 20)
15 nM oligonucleotide labeled with intercalator pigment (YO-NV-S-G, SEQ ID. No. 5; labeled with the intercalator fluorescent pigment between the 12th "C" and 13th "A" from the 5' end, and the hydroxyl group on its 3' end being modified with a glycol group.)
13% DMSO

### Distilled water for adjusting volume

(6) After incubating the aforementioned reaction solution at 43°C for 2 minutes, 5 µL of an enzyme solution having the composition indicated below and pre-heated for 2 minutes at 43°C were added.

### Composition of Enzyme Solution (values shown indicate the values for a final reaction solution volume of 30 µL)

2.0% sorbitol
3.6 µg bovine serum albumin
142 U T7 RNA polymerase (Invitrogen)
6.4 U AMV reverse transcriptase (LifeScience)

### Distilled water for adjusting volume

(7) Subsequently, the reaction solution in each of the PCR tubes was measured, over time, at an excitation wavelength of 470 nm and fluorescent wavelength of 520 nm, while being incubated at 43°C, using a fluorescent spectrophotometer equipped with a temperature control function and capable of directly measuring the PCR tubes.
(8) The results for the rise time (the time required for the ratio in the fluorescence increase to reach 1.2 times the sum of the negative control sample's average value plus 3 standard deviations) of the norovirus artificial standard RNA are shown in Table 3. Four standard RNA samples of GI were shown to be detected within 30 minutes by using a combination of oligonucleotides of the invention of the present application.

**Table 3**

| Artificial RNA Abbreviation | GeneGroup (GI or GII) | Rise Time [min.] |
|---|---|---|
| NV1ART-C | GI | 12.3 |
| NV1ART-D | GI | 24.2 |
| NV1ART-N | GI | 13.1 |
| NV1ART-S | GI | 11.4 |

Table 3 shows the results of measuring norovirus artificial standard RNA (four samples for GI) using the combinations of oligonucleotides used in Example 2. The combinations of oligonucleotides used in Example 2 detected the four standard RNA samples of GI within 30 minutes.

### Industrial Applicability

As has been explained above, the detection method of the invention of the present application is effective for detecting all GI subtypes of norovirus rapidly and with high sensitivity. The oligonucleotide of the invention of the present application is not limited to that of the base sequences shown in the sequence listings (having 18 to 23 bases), but rather can be a nucleotide comprised of at least 10 contiguous bases in those sequences. This is because it is clear that a base sequence of about 10 bases is sufficient for ensuring specificity to a target nucleic acid of a primer or probe under comparatively low-temperature (preferably 43°C) conditions.

It will be appreciated by those skilled in the art that while the invention has been described above in connection with particular embodiments and examples, the invention is not necessarily so limited and that numerous other embodiments, examples, uses, modifications and departures from the embodiments, examples and use may be made without departing from the inventive scope of this application.

## Claims

1. A detection method for detecting norovirus of the genogroup I (GI), using an RNA amplification process comprising the steps of:
producing a cDNA with an RNA-dependent DNA polymerase using a specific sequence of norovirus genome RNA as a template, as well as a first primer having a sequence homologous to said specific sequence, and a second primer having a sequence complementary to said specific sequence, thereby forming a double-strand RNA-DNA, wherein either the first primer or the second primer has a sequence in which a promoter sequence of an RNA polymerase has been added to its 5' end;
degrading the RNA portion of said double-strand RNA-DNA by ribonuclease H, thereby producing a single-strand DNA; and
producing a double-strand DNA having said promoter sequence capable of transcribing the RNA composed of the specific sequence of the RNA or the sequence complementary to said specific sequence of the RNA with a DNA-dependent DNA polymerase using said single-strand DNA as a template;
wherein, the double-strand DNA produces an RNA transcription product in the presence of the RNA polymerase, and said RNA transcription product serves as a template for the subsequent cDNA synthesis with the RNA-dependent DNA polymerase;
**characterized in that**
the first primer is an oligonucleotide consisting of at least the sequence shown in SEQ. ID No. 1; and
the second primer is an oligonucleotide consisting of at least the sequence shown in SEQ. ID No. 4 or the sequence shown in SEQ. ID No. 20.

2. The detection method according to claim 1 wherein the second primer is an oligonucleotide consisting of the sequence shown in SEQ. ID No. 4 or the sequence shown in SEQ. ID No. 20.

3. The detection method according to claim 1 or 2, wherein the RNA amplification process is carried out in the presence of an oligonucleotide that has been labeled with an intercalator fluorescent pigment, and the detection of norovirus is carried out by measuring the fluorescent intensity of the reaction solution; wherein the sequence of said oligonucleotide is complementary to at least a portion of the sequence of the RNA transcription product, and in the situation where complementary binding of said oligonucleotide to said RNA transcription product occurs, the fluorescent properties of the reaction solution change in comparison with the situation where no complex is formed.

4. The detection method according to claim 3 wherein the oligonucleotide labeled with the intercalator fluorescent pigment consists of at least the sequence shown in SEQ. ID No. 5.

## Patentansprüche

1. Nachweisverfahren für den Nachweis von Noroviren der Gengruppe I (GI) unter Verwendung eines RNA-Amplifikationsprozesses mit den Schritten:
Herstellung einer cDNA mit einer RNA-abhängigen DNA-Polymerase unter Verwendung einer speziellen Sequenz von Noroviren-Genom-RNA als Template sowie eines ersten Primers mit einer zu der speziellen Sequenz homologen Sequenz und eines zweiten Primers mit einer zu der speziellen Sequenz komplementären Sequenz, wodurch ein RNA-DNA-Doppelstrang gebildet wird, worin entweder der erste Primer oder der zweite Primer eine Sequenz besitzt, in der eine Promotorsequenz einer RNA-Polymerase zu ihrem 5'-Ende hinzugefügt worden ist;
Abbau des RNA-Anteils des RNA-DNA-Doppelstrangs durch Ribonuklease H, wodurch eine einzelsträngige DNA gebildet wird; und
Herstellung einer doppelsträngigen DNA mit der genannten Promotorsequenz, die zur Transkription der RNA befähigt ist, die aus der speziellen Sequenz der RNA oder der zu der speziellen Sequenz der RNA komplementären Sequenz besteht, mit einer DNA-abhängigen DNA-Polymerase unter Verwendung der genannten einzelsträngigen DNA als Template;
worin die doppelsträngige DNA in Gegenwart von RNA-Polymerase ein RNA-Transkriptionsprodukt liefert und das RNA-Transkriptionsprodukt als Template für die nachfolgende cDNA-Synthese mit der RNA-abhängigen DNA-Polymerase dient;
**dadurch gekennzeichnet, dass**
der erste Primer ein Oligonukleotid ist, das aus zumindest der in SEQ ID Nr. 1 gezeigten Sequenz besteht; und
der zweite Primer ein Oligonukleotid ist, das aus zumindest der in SEQ ID Nr. 4 oder der in SEQ ID Nr. 20 gezeigten Sequenz besteht.

2. Nachweisverfahren nach Anspruch 1, **dadurch gekennzeichnet, dass** der zweite Primer ein Oligonukleotid ist, das aus der in SEQ ID Nr. 4 oder der in SEQ ID Nr. 20 gezeigten Sequenz besteht.

3. Nachweisverfahren nach Anspruch 1 oder 2, **dadurch gekennzeichnet, dass** der RNA-Amplifikationsprozess in Gegenwart eines Oligonukleotids ausgeführt wird, das mit einem fluoreszierenden Interkalator-Pigment markiert worden ist, und der Nachweis der Noroviren ausgeführt wird, indem die Fluoreszenzintensität der Reaktionslösung gemessen wird; wobei die Sequenz des Oligonukleotids zu zumindest einem Abschnitt der Sequenz des RNA-Transkriptionsprodukts komplementär ist, und wodurch sich in der Situation, in der eine komplementäre Bindung des Oligonukleotids an das RNA-Transkriptionsprodukt stattfindet, die Fluoreszenzeigenschaften der Reaktionslösung im Vergleich zu der Situation, in der kein Komplex gebildet wird, ändern.

4. Nachweisverfahren nach Anspruch 3, **dadurch gekennzeichnet, dass** das mit dem fluoreszierenden Interkalator-Pigment markierte Oligonukleotid aus zumindest der in SEQ ID Nr. 5 gezeigten Sequenz besteht.

## Revendications

1. Procédé de détection destiné à détecter un norovirus du génogoupe I (GI), utilisant un procédé d'amplification d'ARN comprenant les étapes consistant à:
produire un ADNc avec une ADN polymérase dépendante d'un ARN en utilisant une séquence spécifique d'un ARN du génome de norovirus comme une matrice, de même qu'une première amorce ayant une séquence homologue à ladite séquence spécifique, et une seconde amorce ayant une séquence complémentaire à ladite séquence spécifique, formant ainsi un ARN-ADN double brin, dans lequel soit la première amorce soit la seconde amorce possède une séquence dans laquelle une séquence de promoteur d'une ARN polymérase a été ajoutée à son extrémité 5' ;
dégrader la partie ARN dudit ARN-ADN double brin avec une ribonucléase H, produisant ainsi un ADN simple brin ; et
produire un ADN double brin ayant ladite séquence de promoteur capable de transcrire l'ARN composé de la séquence spécifique de l'ARN ou de la séquence complémentaire à ladite séquence spécifique de l'ARN, avec une ADN polymérase dépendante d'un ADN en utilisant ledit ADN simple brin comme une matrice ;
dans lequel l'ADN double brin produit un produit de transcription ARN en présence de l'ARN polymérase, et ledit produit de transcription ARN sert de matrice pour la synthèse d'ADNc ultérieure avec l'ADN polymérase dépendante d'un ARN ;
**caractérisé en ce que**
la première amorce est un oligonucléotide consistant en au moins la séquence montrée dans SEQ ID N°1 ; et
la seconde amorce est un oligonucléotide consistant en au moins la séquence montrée dans SEQ ID N°4 ou la séquence montrée dans SEQ ID N°20.

2. Procédé de détection selon la revendication 1, dans lequel la seconde amorce est un oligonucléotide consistant en la séquence montrée dans SEQ ID N°4 ou la séquence montrée dans SEQ ID N°20.

3. Procédé de détection selon la revendication 1 ou 2, dans lequel le procédé d'amplification d'ARN est réalisé en présence d'un oligonucléotide qui a été marqué avec un pigment fluorescent intercalant, et la détection du norovirus est réalisée en mesurant l'intensité de fluorescence de la solution de réaction; dans lequel la séquence dudit oligonucléotide est complémentaire à au moins une partie de la séquence du produit de transcription ARN, et, dans la situation dans laquelle la liaison complémentaire dudit oligonucléotide audit produit de transcription ARN se produit, les propriétés fluorescentes de la solution de réaction changent en comparaison avec la situation dans laquelle aucun complexe ne se forme.

4. Procédé de détection selon la revendication 3, dans lequel l'oligonucléotide marqué avec le pigment fluorescent intercalant consiste en au moins la séquence montrée dans SEQ ID N°5.


### SEQUENCE LISTING

<110> TOSOH Corporation
   <120> Norovirus Detection Reagent
   <130> R002
<150> JP 2004-027802
   <151> 2004-02-04
<160> 20
<210> 1
   <211> 23
   <212> DNA
   <213> Artificial Sequence
<220>
   <223> NV1-3FM-9-T7
<400> 1
   ttgtggacag gagatcgcta tct 23
<210> 2
   <211> 23
   <212> DNA
   <213> Artificial Sequence
<220>
   <223> NV2-3FM-T7
<400> 2
   acgtgggagg gcgatcgcaa tct 23
<210> 3
   <211> 23
   <212> DNA
   <213> Artificial Sequence
<220>
   <223> NV2-3F20+3-T7
<400> 3
   tgggagggcg atcgcaatct 23
<210> 4
   <211> 18
   <212> DNA
   <213> Artificial Sequence
<220>
   <223> NV2-7RM
   <400> 4
   tctaatccag gggtctat 18
<210> 5
   <211> 18
   <212> DNA
   <213> Artificial Sequence
<220>
   <223> YO-NV-S-G
<400> 5
   gacgccatct tcatttac 18
<210> 6
   <211> 90
   <212> RNA
   <213> Artificial Sequence
<220>
   <223> NV1ART-C
   <400> 6
   gaauacaacg cuuccaugau cucggauugu ggacaggaga ucgcaaucuc cguaaaugau 60
   gauggcgucu gauugauccc uggauaauca 90
<210> 7
   <211> 90
   <212> RNA
   <213> Artificial Sequence
<220>
   <223> NV1ART-D
   <400> 7
   gaauacaacg cuuccaugau cugagcaugu ggacagggga ucgcgaucuc uguaaaugau 60
   gauggcgucu gauugacccc uggauaauga 90
<210> 8
   <211> 90
   <212> RNA
   <213> Artificial Sequence
<220>
   <223> NV1ART-N
   <400> 8
   gaauacaacg cuuccaugac cucggauugu ggacaggaga ucgcgaucuu cguaaaugau 60
   gauggcgucu uauugauccc uggauaauua 90
<210> 9
   <211> 90
   <212> RNA
   <213> Artificial Sequence
<220>
   <223> NV1ART-S
   <400> 9
   gaauacaacg guuccaugac cuugguuugu ggacaggaga ucgcaaucuc uguaaaugau 60
   gauggcgucu gauugauccc uggauuguua 90
<210> 10
   <211> 90
   <212> RNA
   <213> Artificial Sequence
<220>
   <223> NV2ART-C
   <400> 10
   gaauacaaag auucucagau cugagcacgu gggagggcga ucgcaaucug ugugaaugaa 60
   gauggcgucg aauugacccc uggauuagaa 90
<210> 11
   <211> 90
   <212> RNA
   <213> Artificial Sequence
<220>
   <223> NV2ART-H
   <400> 11
   gaauacaaag auucucagac cugagcacgu gggagggcga ucgcaaucug ugugaaugaa 60
   gauggcgucg gauagacccc uggauuagaa 90
<210> 12
   <211> 90
   <212> RNA
   <213> Artificial Sequence
<220>
   <223> NV2ART-M
<400> 12
   gaauacaaag guucucagau cuaagcacau gggagggcga ucgcaaucug ugugaaugaa 60
   gauggcgucg aauugauccc uggauuauga 90
<210> 13
   <211> 90
   <212> RNA
   <213> Artificial Sequence
<220>
   <223> NV2ART-S
   <400> 13
   gaauacaaag auucucggau uugagcacgu gggagggcga ucgcaaucuu ugugaaugaa 60
   gauggcgucg uauagacccu uggauuagag 90
<210> 14
   <211> 24
   <212> DNA
   <213> Artificial Sequence
<220>
   <223> NV1-3SM-9
<400> 14
   ccacaatccg agatcatgga agcg 24
<210> 15
   <211> 24
   <212> DNA
   <213> Artificial Sequence
<220>
   <223> NV2-3SM
<400> 15
   ccacgtgctc agatctgaga atct 24
<210> 16
   <211> 20
   <212> DNA
   <213> Artificial Sequence
<220>
   <223> NV2-3S20+3
<400> 16
   ctcccatgtg cttaagtccg 20
<210> 17
   <211> 51
   <212> DNA
   <213> Artificial Sequence
<220>
   <223> NV1-3FM-9
<400> 17
   aattctaata cgactcacta tagggagatt gtggacagga gatcgctatc t 51
<210> 18
   <211> 51
   <212> DNA
   <213> Artificial Sequence
<220>
   <223> NV2-3FM
<400> 18
   aattctaata cgactcacta tagggagaac gtgggagggc gatcgcaatc t 51
<210> 19
   <211> 48
   <212> DNA
   <213> Artificial Sequence
<220>
   <223> NV2-3F20+3
<400> 19
   aattctaata cgactcacta tagggagatg ggagggcgat cgcaatct 48
<210> 20
   <211> 18
   <212> DNA
   <213> Artificial Sequence
<220>
   <223> NV1-7RM18-1
<400> 20
   gattatccag ggatcaat 18
<220>
   <223> NV2-3F20+3-T7
<400> 3
   tgggagggcg atcgcaatct 20
<210> 4
   <211> 18
   <212> DNA
   <213> Artificial Sequence
<220>
   <223> NV2-7RM
   <400> 4
   tctaatccag gggtctat 18
<210> 5
   <211> 18
   <212> DNA
   <213> Artificial Sequence
<220>
   <223> YO-NV-S-G
<400> 5
   gacgccatct tcatttac 18
<210> 6
   <211> 90
   <212> RNA
   <213> Artificial Sequence
<220>
   <223> NV1ART-C
   <400> 6
   gaauacaacg cuuccaugau cucggauugu ggacaggaga ucgcaaucuc cguaaaugau 60
   gauggcgucu gauugauccc uggauaauca 90

### SEQUENCE LISTING

<110> TOSOH Corporation
   <120> Norovirus Detection Reagent
   <130> R002
<150> JP 2004-027802
   <151> 2004-02-04
<160> 20
<210> 1
   <211> 23
   <212> DNA
   <213> Artificial Sequence
<220>
   <223> NV1-3FM-9-T7
<400> 1
   ttgtggacag gagatcgcta tct 23
<210> 2
   <211> 23
   <212> DNA
   <213> Artificial Sequence
<220>
   <223> NV2-3FM-T7
<400> 2
   acgtgggagg gcgatcgcaa tct 23
<210> 3
   <211> 23
   <212> DNA
   <213> Artificial Sequence
<220>
   <223> NV2-3F20+3-T7
<400> 3
   tgggagggcg atcgcaatct 23
<210> 4
   <211> 18
   <212> DNA
   <213> Artificial Sequence
<220>
   <223> NV2-7RM
   <400> 4
   tctaatccag gggtctat 18
<210> 5
   <211> 18
   <212> DNA
   <213> Artificial Sequence
<220>
   <223> YO-NV-S-G
<400> 5
   gacgccatct tcatttac 18
<210> 6
   <211> 90
   <212> RNA
   <213> Artificial Sequence
<220>
   <223> NV1ART-C
   <400> 6
   gaauacaacg cuuccaugau cucggauugu ggacaggaga ucgcaaucuc cguaaaugau   60
   gauggcgucu gauugauccc uggauaauca                                     90
<210> 7
   <211> 90
   <212> RNA
   <213> Artificial Sequence
<220>
   <223> NV1ART-D
   <400> 7
   gaauacaacg cuuccaugau cugagcaugu ggacagggga ucgcgaucuc uguaaaugau   60
   gauggcgucu gauugacccc uggauaauga                                     90
<210> 8
   <211> 90
   <212> RNA
   <213> Artificial Sequence
<220>
   <223> NV1ART-N
   <400> 8
   gaauacaacg cuuccaugac cucggauugu ggacaggaga ucgcgaucuu cguaaaugau   60
   gauggcgucu uauugauccc uggauaauua                                     90
<210> 9
   <211> 90
   <212> RNA
   <213> Artificial Sequence
<220>
   <223> NV1ART-S
   <400> 9
   gaauacaacg guuccaugac cuugguuugu ggacaggaga ucgcaaucuc uguaaaugau   60
   gauggcgucu gauugauccc uggauuguua                                     90
<210> 10
   <211> 90
   <212> RNA
   <213> Artificial Sequence
<220>
   <223> NV2ART-C
   <400> 10
   gaauacaaag auucucagau cugagcacgu gggagggcga ucgcaaucug ugugaaugaa 60
   gauggcgucg aauugacccc uggauuagaa 90
<210> 11
   <211> 90
   <212> RNA
   <213> Artificial Sequence
<220>
   <223> NV2ART-H
   <400> 11
   gaauacaaag auucucagac cugagcacgu gggagggcga ucgcaaucug ugugaaugaa 60
   gauggcgucg gauagacccc uggauuagaa 90
<210> 12
   <211> 90
   <212> RNA
   <213> Artificial Sequence
<220>
   <223> NV2ART-M
<400> 12
   gaauacaaag guucucagau cuaagcacau gggagggcga ucgcaaucug ugugaaugaa 60
   gauggcgucg aauugauccc uggauuauga 90
<210> 13
   <211> 90
   <212> RNA
   <213> Artificial Sequence
<220>
   <223> NV2ART-S
   <400> 13
   gaauacaaag auucucggau uugagcacgu gggagggcga ucgcaaucuu ugugaaugaa 60
   gauggcgucg uauagacccu uggauuagag 90
<210> 14
   <211> 24
   <212> DNA
   <213> Artificial Sequence
<220>
   <223> NV1-3SM-9
<400> 14
   ccacaatccg agatcatgga agcg 24
<210> 15
   <211> 24
   <212> DNA
   <213> Artificial Sequence
<220>
   <223> NV2-3SM
<400> 15
   ccacgtgctc agatctgaga atct 24
<210> 16
   <211> 20
   <212> DNA
   <213> Artificial Sequence
<220>
   <223> NV2-3S20+3
<400> 16
   ctcccatgtg cttaagtccg 20
<210> 17
   <211> 51
   <212> DNA
   <213> Artificial Sequence
<220>
   <223> NV1-3FM-9
<400> 17
   aattctaata cgactcacta tagggagatt gtggacagga gatcgctatc t 51
<210> 18
   <211> 51
   <212> DNA
   <213> Artificial Sequence
<220>
   <223> NV2-3FM
<400> 18
   aattctaata cgactcacta tagggagaac gtgggagggc gatcgcaatc t 51
<210> 19
   <211> 48
   <212> DNA
   <213> Artificial Sequence
<220>
   <223> NV2-3F20+3
<400> 19
   aattctaata cgactcacta tagggagatg ggagggcgat cgcaatct 48
<210> 20
   <211> 18
   <212> DNA
   <213> Artificial Sequence
<220>
   <223> NV1-7RM18-1
<400> 20
   gattatccag ggatcaat 18